# EUROPEAN PATENT APPLICATION

(11) **EP 4 729 021 A1**
(43) Date of publication of application: **22.04.2026**
(21) Application number: 24823742.2
(22) Date of filing: 14.06.2024
(51) Int. Cl.: A61C 7/00, A61C 9/00, G06T 1/00, G06T 19/20, G06T 17/00, G06T 7/90, G16H 50/50, G16H 30/00, A61B 5/00, A61C 13/00

(54) **ORAL IMAGE PROCESSING DEVICE, ORAL IMAGE PROCESSING METHOD, AND COMPUTER-READABLE RECORDING MEDIUM**

(30) Priority: 15.06.2023 KR 20230077004
(71) Applicant: Medit Corp., Seoul 07207 (KR)
(72) Inventor: LEE, Sung Hoon, Seoul 07207 (KR); KIM, Jin Young, Seoul 07207 (KR)
(74) Representative: Kim Kang, Jae Hee
(86) International application number: PCT/KR2024/008180
(87) International publication number: WO 2024/258225

(57) **Abstract**

The present disclosure provides an oral image processing apparatus and an oral image processing method. In an embodiment, the oral image processing method may comprise the steps of: acquiring an oral image including a plurality of teeth; acquiring a selection signal for selecting at least one selection point among a plurality of selection points located between respective two adjacent teeth among the plurality of teeth; acquiring a target movement amount of at least one adjacent tooth adjacent to the selected selection point; and moving the at least one adjacent tooth by the target movement amount in a first movement direction which is a direction toward the selected selection point.

## Description

### Technical Field

The disclosure relates to an oral image processing apparatus and an oral image processing method, and specifically, to an oral image processing apparatus and an oral image processing method for moving teeth of an oral image.

### Background Art

Dental computer aided design (CAD)/dental computer aided manufacturing (CAM) technologies are widely used in treatment such as orthodontics. The most important thing in dental treatment using CAD/CAM is to obtain sophisticated three-dimensional (3D) data on the shape of an object such as a patient's teeth, gums, and jawbones. In performing dental treatment, when 3D data obtained from an object is used, there is an advantage that accurate calculation may be performed by a computer. For example, in dental treatment using dental CAD/CAM, methods such as computed tomography (CT), magnetic resonance imaging (MRI), and optical scanning may be used to acquire three-dimensional data of an object.

To determine an appropriate treatment method before proceeding with orthodontic treatment in treatment such as orthodontic treatment, an orthodontic simulation of the tooth shape expected during orthodontic treatment may be provided. Orthodontic simulations may be provided to patients through various scenarios such as interproximal reduction (IPR) (hereinafter referred to as "IPR"), tooth extraction, and tooth movement during orthodontic simulation. In addition, through orthodontic simulation, it is possible to predict the interval between corrected teeth that may occur after orthodontic correction, for example, a intrerproximal interval, such as a black triangle, and adjust the intrerproximal interval by virtually performing an IPR to delete the intrerproximal interval.

### Detailed Description

### Technical Problem

The disclosure provides an oral image processing apparatus that selects a selection point located between two adjacent teeth and moves at least one adjacent tooth by an acquired target movement amount, and a method that performs an operation accordingly.

### Solution to Problem

According to an aspect of the disclosure, an oral image processing method may include acquiring an oral image including a plurality of teeth. The oral image processing method may include acquiring a selection signal for selecting at least one selection point among a plurality of selection points positioned between respective two adjacent teeth among the plurality of teeth. The oral image processing method may include acquiring a target movement amount of at least one adjacent tooth adjacent to the selection point. The oral image processing method may include moving the at least one adjacent tooth by the target movement amount in a first movement direction, which is a direction toward the selected selection point.

In an embodiment, the oral image processing method may further include providing a user input interface capable of inputting a target movement amount. The oral image processing method may acquire a target movement amount through the user input interface.

In an embodiment, the oral image processing method may include selecting, as a target tooth to be moved, at least one tooth positioned in a direction from the at least one selection point selected based on the at least one adjacent tooth toward the at least one adjacent tooth. The oral image processing method may include moving the target tooth to be moved in a direction toward at least one adjacent tooth.

In an embodiment, the at least one adjacent tooth may include at least one of a first adjacent tooth adjacent in a distal direction or a second adjacent tooth adjacent in a mesial direction from the selected at least one selection point.

In an embodiment, the oral image processing method may include selecting, as a target tooth to be moved, a first mobile tooth located in the distal direction based on the first adjacent tooth when the at least one adjacent tooth includes the first adjacent tooth. The oral image processing method may include selecting, as a target tooth to be moved, a second mobile tooth located in the mesial direction to the dental midline based on the second adjacent tooth when the at least one adjacent tooth includes the second adjacent tooth. The oral image processing method may include selecting, as target teeth to be moved, a first mobile tooth and a second mobile tooth when the at least one adjacent tooth includes the first adjacent tooth and the second adjacent tooth.

In an embodiment, the oral image processing method may further include moving the first adjacent tooth by a first movement amount to the selected at least one selection point and moving the second adjacent tooth by a second movement amount to the selected at least one selection point when the at least one adjacent tooth includes the first adjacent tooth and the second adjacent tooth. A sum of the first movement amount and the second movement amount may be equal to the target movement amount.

In an embodiment, the ratio of the first movement amount to the second movement amount may be a preset ratio, or the ratio of the first movement amount to the second movement amount may be proportional to a distance from the dental midline to the at least one selected selection point.

In an embodiment, the oral image processing method may include displaying a color of at least one adjacent tooth among the plurality of teeth and a color of a target tooth to be moved to be distinguished from the colors of the remaining teeth among the plurality of teeth.

In an embodiment, the oral image processing method may include acquiring a dental arch line corresponding to the plurality of teeth from the oral image. The oral image processing method may include moving the at least one adjacent tooth moved in the first movement direction in a second movement direction, which is a direction toward the dental arch line.

In an embodiment, the oral image processing method may repeat moving the at least one adjacent tooth in the first movement direction and moving the at least one adjacent tooth in the second movement direction so that the at least one adjacent tooth may be positioned close to the dental arch line.

According to an aspect of the disclosure, an oral image processing apparatus may include a memory storing at least one instruction and at least one processor executing the at least one instruction stored in the memory. The at least one processor may acquire an oral image including a plurality of teeth. The at least one processor may acquire a selection signal for selecting at least one selection point among a plurality of selection points positioned between respective two adjacent teeth among a plurality of teeth. The at least one processor may acquire a target movement amount of at least one adjacent tooth adjacent to the selected selection point. The at least one processor may move the at least one adjacent tooth by the target movement amount in a first movement direction, which is a direction toward the selected selection point.

In an embodiment, the at least one processor may provide a user with a user input interface capable of inputting a target movement amount. The at least one processor may acquire a target movement amount through the user input interface.

In an embodiment, the at least one processor may select, as a target tooth to be moved, at least one tooth positioned in a direction from the at least one selection point selected based on the at least one adjacent tooth toward the at least one adjacent tooth. The at least one processor may move the target tooth to be moved toward at least one adjacent tooth.

In an embodiment, the at least one adjacent tooth may include at least one of a first adjacent tooth adjacent in a distal direction or a second adjacent tooth adjacent in a mesial direction from the selected at least one selection point.

In an embodiment, when at least one adjacent tooth includes a first adjacent tooth, the at least one processor may select, as a target tooth to be moved, a first mobile tooth located in the distal direction based on the first adjacent tooth. When at least one adjacent tooth includes a second adjacent tooth, the at least one processor may select, as a target tooth to be moved, a second mobile tooth located in a mesial direction to the dental midline based on the second adjacent tooth. When at least one adjacent tooth includes a first adjacent tooth and a second adjacent tooth, the at least one processor may select, as target teeth to be moved, a first mobile tooth and a second mobile tooth.

In an embodiment, when at least one adjacent tooth includes a first adjacent tooth and a second adjacent tooth, the at least one processor may move the first adjacent tooth to the selected at least one selection point by a first movement amount. The at least one processor may move the second adjacent tooth to the selected at least one selection point by a second movement amount. A sum of the first movement amount and the second movement amount may be equal to the target movement amount.

In an embodiment, the ratio of the first movement amount to the second movement amount may be a preset ratio, or the ratio of the first movement amount to the second movement amount may be proportional to a distance from the dental midline to the at least one selected selection point.

In an embodiment, the at least one processor may acquire a dental arch line corresponding to the plurality of teeth from the oral image. The at least one processor may move the at least one adjacent tooth moved in the first movement direction in a second movement direction, which is a direction toward the dental arch line.

In an embodiment, the at least one processor may repeat moving the at least one adjacent tooth in the first movement direction and moving the at least one adjacent tooth in the second movement direction so that the at least one adjacent tooth may be positioned close to the dental arch line.

According to an aspect of the disclosure, a non-transitory computer-readable recording medium having recorded thereon at least one instruction to be executed by at least one processor of an oral image processing apparatus, wherein, when the at least one instruction is executed by the at least one processor of the oral image processing apparatus, the oral image processing apparatus may acquire an oral image including a plurality of teeth, acquire a selection signal for selecting at least one selection point among a plurality of selection points located between respective two adjacent teeth among a plurality of teeth, acquire a target movement amount of at least one adjacent tooth adjacent to the selected selection point, and move the at least one adjacent tooth by the target movement amount in a first movement direction toward the selected selection point.

### Advantageous Effects of Invention

The oral image processing apparatus and the oral image processing method according to the disclosed embodiment may move at least one adjacent tooth adjacent to an identified selection point by a target movement amount in a direction of the identified selection point. Accordingly, it is possible to acquire an oral image after moving at least one adjacent tooth by a target movement amount toward the identified selection point.

The oral image processing apparatus and the oral image processing method according to the disclosed embodiment may increase user convenience by providing a tooth model according to a target tooth deletion amount.

### Brief Description of Drawings

The present disclosure may be easily understood as a combination of the following detailed description and accompanying drawings, and reference numerals refer to structural elements.
FIG. 1 is a diagram for describing an oral image processing apparatus and method according to an embodiment of the disclosure.
FIG. 2 is a block diagram for describing an oral image processing apparatus according to an embodiment of the disclosure.
FIG. 3 is a flowchart for describing an oral image processing method according to an embodiment of the disclosure.
FIG. 4 is a diagram for describing a plurality of teeth identified from an oral image according to an embodiment of the disclosure.
FIG. 5 is a flowchart for describing a method of calculating a plurality of selection points positioned between respective two adjacent teeth according to an embodiment of the disclosure.
FIG. 6 is a diagram for describing a method of generating respective bounding boxes of two adjacent teeth according to an embodiment of the disclosure.
FIG. 7 is a diagram for describing a method of calculating center points of respective bounding boxes and generating a connection line connecting the center points according to an embodiment of the disclosure.
FIG. 8 is a diagram for describing a method for calculating a contact point and a selection point where a connection line and a mesh of two teeth meet, according to an embodiment of the disclosure.
FIG. 9A is a diagram for describing selection of one selection point from among a plurality of selection points according to an embodiment of the disclosure.
FIG. 9B is a diagram for describing selection of two or more selection points from among a plurality of selection points according to an embodiment of the disclosure.
FIG. 10 is a flowchart for describing a method of selecting a target tooth to be moved and moving the selected target tooth to be moved, according to an embodiment of the disclosure.
FIG. 11A is a diagram for describing selecting a first adjacent tooth and a target tooth to be moved corresponding to the first adjacent tooth according to an embodiment of the disclosure.
FIG. 11B is a diagram for describing selecting a second adjacent tooth and a target tooth to be moved corresponding to the second adjacent tooth according to an embodiment of the disclosure.
FIG. 11C is a diagram for describing selecting a first adjacent tooth and a second adjacent tooth and a target tooth to be moved corresponding to the first adjacent tooth and the second adjacent tooth, according to an embodiment of the disclosure.
FIG. 12 is a flowchart for describing a movement amount of each of a first adjacent tooth and a second adjacent tooth when a first adjacent tooth and a second adjacent tooth are selected according to an embodiment of the disclosure.
FIG. 13 is a diagram for describing a target movement amount of at least one adjacent tooth according to an embodiment of the disclosure.
FIG. 14 is a diagram for describing moving at least one adjacent tooth to an identified selection point by a target movement amount, according to an embodiment of the disclosure.
FIG. 15 is a flowchart for describing moving at least one adjacent tooth to a dental arch line according to an embodiment of the disclosure.
FIG. 16 is a diagram for describing a direction in which at least one adjacent tooth is moved to a dental arch line according to an embodiment of the disclosure.
FIG. 17 is a diagram for describing that at least one adjacent tooth is moved to a dental arch line according to an embodiment of the disclosure.

### Mode for the Invention

The disclosure will be described more fully hereinafter with reference to the accompanying drawings, in order to clarify the scope of the present disclosure, explain the principle of the present disclosure, and disclose embodiments so that a person with ordinary skill in the art may implement the present disclosure. The disclosed embodiments may be implemented in various forms.

Throughout the specification, the same reference numerals refer to the same components. The disclosure does not describe all elements of embodiments, and general description in the technical field to which the present disclosure belongs or redundant description between the embodiments are omitted. The term "part" or "portion" used in the disclosure may be implemented as software or hardware, and according to embodiments, a plurality of "parts" or "portions" may be implemented as a single unit or element, or one "part" or "portion" may include a plurality of units or elements. Hereinafter, the operating principles and embodiments of the present disclosure are described with reference to the accompanying drawings.

In the disclosure, an image may include an image (hereinafter, referred to as an "oral image") representing at least one tooth or an oral cavity including at least one tooth.

In addition, in this disclosure, an image may be a two-dimensional image of an object or a three-dimensional model or a three-dimensional image representing an object in three dimensions. In addition, in this disclosure, an image may mean data necessary to represent an object in two or three dimensions, for example, raw data acquired from at least one image sensor. Specifically, raw data is data acquired to generate an image, and may be data (e.g., two-dimensional data) acquired from at least one image sensor included in a three-dimensional scanner when scanning an object using a three-dimensional scanner.

In the disclosure, the "oral image" may include various polygonal meshes. For example, when two-dimensional data is acquired using an oral scanner, a data processing apparatus may calculate coordinates of multiple illuminated surface points using triangulation methods. Coordinates of surface points may be accumulated as the amount of scan data increases by scanning the surface of an object while moving along the surface of the object using an oral scanner. As a result of the image acquisition, a point cloud of vertices may be identified to indicate a range of the surface. Points in the point cloud may represent actual measured points on the three-dimensional surface of the object. A surface structure may be approximated by forming a polygonal mesh in which adjacent vertices of the point cloud are connected by a line segment. The polygonal mesh may be variously determined, such as a triangle, a square, a pentagonal mesh, etc. The relationship between a polygons and a neighboring polygon of such a mesh model may be used to extract features of tooth boundaries, such as curvature, minimum curvature, edge, spatial relationships, and the like.

Hereinafter, embodiments are described in detail with reference to the drawings.

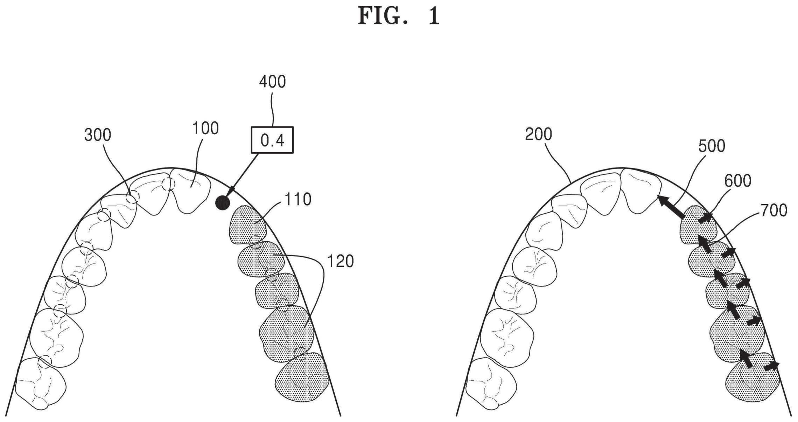

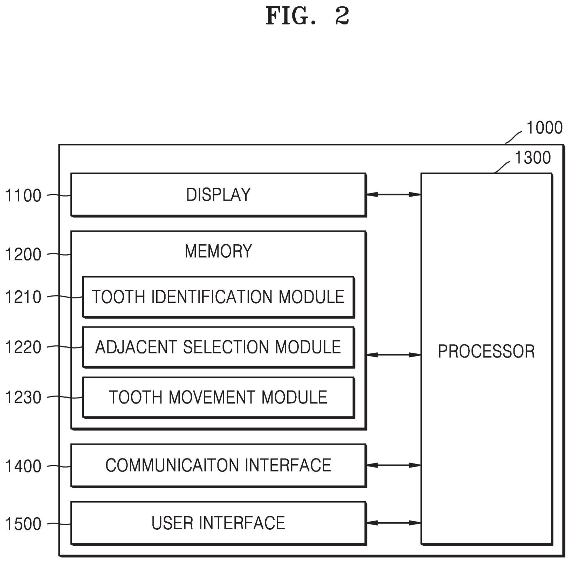

FIG. 1 is a diagram for describing an oral image processing apparatus and method according to an embodiment of the disclosure. FIG. 2 is a block diagram for describing an oral image processing apparatus according to an embodiment of the disclosure.

Referring to FIGS. 1 and 2, the oral image processing method according to the disclosure may be performed by an oral image processing apparatus 1000. In an embodiment, the oral image processing apparatus 1000 may acquire an oral image including a plurality of teeth 100, 110, and 120. In an embodiment, the oral image processing apparatus 1000 may obtain an oral image from a 3D scanner.

In an embodiment, the oral image may be an image generated by a 3D scanner that scans an object including at least one of a human oral cavity, an artificial structure, or a plaster model modeled after the oral cavity or the artificial structure. In an embodiment, the 3D scanner may include a handheld oral scanner in which a user holds and moves by hand and scans an object. In addition, the 3D scanner may include a table-type scanner that scans an object using the rotation of a table. However, the present disclosure is not limited thereto, and the 3D scanner may include a scanner having various shapes capable of acquiring an oral image.

However, the present disclosure is not limited thereto, and the oral image processing apparatus 1000 may acquire a pre-generated oral image from a cloud server or an external storage device.

In an embodiment, the oral image processing apparatus 1000 may include a display 1100. The oral image processing apparatus 1000 may display, on the display 1100, a plurality of teeth 100, 110, and 120, gingiva, a dental arch line 200, and the like identified based on the acquired oral image to provide the same to a user using the oral image processing apparatus 1000.

In an embodiment, the oral image processing apparatus 1000 may display, on the display, an image for each operation in the process of processing the oral image 1100 and provide the same to the user.

In an embodiment, the oral image processing apparatus 1000 may display, on the display 1100, a plurality of selection points 300, a selected selection point, at least one adjacent tooth 110, and a target tooth 120 to be moved and provide the same to the user.

In an embodiment, the oral image processing apparatus 1000 may display a target movement amount of at least one adjacent tooth on the display 1100 and provide the same to the user.

In an embodiment, the oral image processing apparatus 1000 may display, on the display 1100, an image in which the at least one adjacent tooth 110 is moved to at least one identified selection point by a target movement amount and provide the same to the user.

In an embodiment, after the at least one adjacent tooth 110 is moved, the oral image processing apparatus 1000 may display, on the display 1100, an image which is acquired by moving a target tooth 120 to be moved in a direction toward the at least one adjacent tooth 110 and provide the same to the user.

Hereinafter, an operation of the oral image processing apparatus 1000 for each operation is described.

In an embodiment, the oral image processing apparatus 1000 may identify a plurality of teeth 100, 110, and 120 from an oral image. In FIG. 1, twelve (12) teeth are identified from an oral image, but the present disclosure is not limited thereto.

In addition, the oral image processing apparatus 1000 may identify gingiva and a dental arch line 200 as well as a plurality of teeth 100, 110, and 120 from the oral image. In an embodiment, the dental arch line may mean an arch-shaped structure in which the plurality of teeth 100, 110, and 120 placed on each of the maxilla and the mandible are arranged.

In an embodiment, the dental arch line 200 may be a line corresponding to the dental arch. In an embodiment, the dental arch line 200 may be a line in contact with the plurality of teeth 100, 110, and 120 arranged according to the dental arch. However, the present disclosure is not limited thereto. The dental arch line 200 may be arbitrarily set. The dental arch line 200 may be set as a line for arranging the plurality of teeth 100, 110, and 120.

In an embodiment, the oral image processing apparatus 1000 may calculate a plurality of selection points 300 positioned between respective two adjacent teeth among the plurality of teeth 100, 110, and 120.

In an embodiment, the oral image processing apparatus 1000 may acquire a selection signal including information for selecting one selection point from among the plurality of selection points 300. In an embodiment, as one selection point is selected from among a plurality of selection points 300, the oral image processing apparatus 1000 may generate a selection signal.

In an embodiment, when one selection point is selected from among a plurality of selection points 300 through a user interface (UI) provided to a user, the oral image processing apparatus 1000 may acquire a selection signal. In an embodiment, when a user clicks and selects one of the plurality of selection points 300 through a mouse cursor, the oral image processing apparatus 1000 may acquire a selection signal.

The present disclosure is not limited thereto, and the oral image processing apparatus 1000 may acquire a selection signal including information for selecting two or more selection points from among the plurality of selection points 300. In an embodiment, when a user drags and selects two or more selection points from among the plurality of selection points 300 using a plurality of mouse cursors, the oral image processing apparatus 1000 may acquire a selection signal including information for selecting two or more selection points.

However, the present disclosure is not limited thereto, and when the display 1100 includes a touch interface, and when the user touches and selects one of the plurality of selection points 300 displayed on the display 1100, the oral image processing apparatus 1000 may acquire a selection signal.

In an embodiment, the oral image processing apparatus 1000 may select at least one adjacent tooth 110 which is adjacent to the at least one selection point based on the selection signal. Although FIG. 1 shows a first adjacent tooth selected in a direction adjacent to a distal direction from at least one selection point among the plurality of teeth 100, 110, and 120, the present disclosure is not limited thereto.

In an embodiment, the oral image processing apparatus 1000 may select, as at least one adjacent tooth 110, a tooth adjacent to at least one selection point in at least one direction of a mesial direction or a distal direction.

In an embodiment, the "at least one adjacent tooth 110" may be a tooth that is moved by a target movement amount to be described later in a direction toward at least one selection point.

In an embodiment, the oral image processing apparatus 1000 may select, as a target tooth 120 to be moved, at least one tooth positioned in a direction toward the at least one adjacent tooth 110 from the at least one selection point based on the at least one adjacent tooth 110.

In an embodiment, the "tooth 120 to be moved" may be a tooth that, after the at least one adjacent tooth 110 is moved toward the at least one selection point, is moved in a direction toward at least one adjacent tooth 110 so as to maintain an interval with the at least one adjacent tooth 110. In an embodiment, when target teeth to be moved include two or more teeth, the target teeth 120 to be moved may be moved to maintain a gap between each other.

In an embodiment, the oral image processing apparatus 1000 may acquire a target movement amount of the at least one adjacent tooth 110. In an embodiment, the "target movement amount" may be a movement amount for moving at least one adjacent tooth 110 in a direction to a selected selection point.

In an embodiment, the oral image processing apparatus 1000 may acquire the target movement amount by an operation of inputting the target movement amount of the at least one adjacent tooth 110.

In an embodiment, when a target movement amount of at least one adjacent tooth 110 is input through a UI provided to a user, the oral image processing apparatus 1000 may acquire the target movement amount. In an embodiment, when a user inputs a target movement amount into an input space through which the user may input a value of the target movement amount or when the user inputs a target movement amount using a sliding bar capable of adjusting the size of the target movement amount, the oral image processing apparatus 1000 may acquire the target movement amount. However, the present disclosure is not limited thereto, and the oral image processing apparatus 1000 may acquire the target movement amount even when the target movement amount is input by another method.

In an embodiment, the oral image processing apparatus 1000 may move at least one adjacent tooth 110 by a target movement amount in a direction 500 toward a selected selection point. Hereinafter, a direction toward the selected selection point will be referred to as a first movement direction 500.

The oral image processing apparatus 1000 may provide a user with an image in which at least one adjacent tooth 110 has moved to a selected selection point by a target movement amount. The user may check an image when the at least one adjacent tooth moves by the target movement amount to the selected selection point.

The user may check the result of interdental movement after inter proximal reduction (IPR) or the result of interdental movement through orthodontic correction through the oral image processing apparatus 1000 and the oral image processing method according to an embodiment, and determine whether to perform inter proximal reduction or orthodontic correction. In addition, the user may check the result of interdental movement after tooth extraction and determine whether to perform tooth extraction through the oral image processing apparatus 1000 and the oral image processing method according to an embodiment.

In an embodiment, the oral image processing apparatus 1000 may move at least one adjacent tooth 110 in a first movement direction 500 by a target movement amount, and then move the at least one adjacent tooth 110 in a direction 600 toward the dental arch line 200. Hereinafter, for convenience of description, a direction toward the dental arch line 200 will be referred to as a second movement direction 600.

In an embodiment, when at least one adjacent tooth 110 is moved by a target movement amount in the first movement direction 500, the at least one adjacent tooth 110 may move away from the dental arch line 200 compared to before movement. Therefore, the oral image processing apparatus 0100 may move the at least one adjacent tooth 110 in the first movement direction 500 and then move the at least one adjacent tooth 110 in the second movement direction 600 so that the at least one adjacent tooth 110 is in contact with the dental arch line 200.

In an embodiment, when the at least one adjacent tooth 110 is moved in the second movement direction 600, the movement amount of the at least one adjacent tooth 110 in the first movement direction 500 may be different from the target movement amount. Accordingly, the oral image processing apparatus 1000 may move the at least one adjacent tooth 110 again in the first movement direction 500.

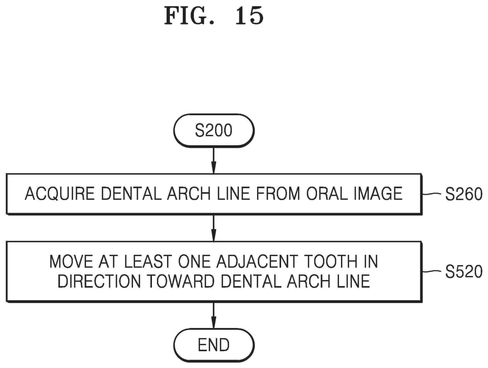

In an embodiment, the oral image processing apparatus 1000 may repeat an operation of moving the at least one adjacent tooth 110 in the first moving direction 500 and an operation of moving the at least one adjacent tooth 110 in the second moving direction 600. Hereinafter, a detailed description will be described with reference to FIGS. 15 to 19.

In an embodiment, the oral image processing apparatus 1000 may move the at least one adjacent tooth 110 in the first movement direction 500 by a target movement amount, and then move the target teeth 120 to be moved in a direction 700 toward the at least one adjacent tooth 110. Accordingly, even if the at least one adjacent tooth 110 is moved by a target movement amount, the gap between the at least one adjacent tooth 110 and the target teeth 120 to be moved may be maintained compared to before the movement.

Hereinafter, the oral image processing apparatus 1000 and the oral image processing method will be described in detail.

Referring to FIG. 2, in an embodiment, the oral image processing apparatus 1000 may include a display 1100, a memory 1200, at least one processor 1300, a communication interface 1400, and a user interface 1500.

In an embodiment, not all of the components illustrated in FIG. 2 are essential components. The oral image processing apparatus 1000 may be implemented by more components than the components shown in FIG. 2, and the oral image processing apparatus 1000 may be implemented by fewer components.

In an embodiment, the display 1100, the memory 1200, the at least one processor 1300, the communication interface 1400, and the user interface 1500 may be electrically and/or physically connected to each other.

In an embodiment, the oral image processing apparatus 1000 may be a computing device such as a smartphone, a laptop computer, a desktop computer, a personal digital assistant (PDA), and a tablet personal computer (PC), but is not limited thereto. In addition, the oral image processing apparatus 1000 may exist in the form of a server (or server device) for processing an oral image.

In an embodiment, the display 1100 may display an oral image. In an embodiment, the display 1100 may display an oral image processed under the control by the at least one processor 1300. In an embodiment, the display 1100 may display images corresponding to an operation of processing an oral image under the control by the at least one processor 1300.

In an embodiment, the display 1100 may display a user interface screen including an oral image processed by the at least one processor 1300. In an embodiment, the display 1100 may display a user interface screen including information related to a patient's dental treatment.

In an embodiment, instructions, program code , or various types of modules for performing functions or operations of the oral image processing apparatus 1000 may be stored in the memory 1200.

In an embodiment, a tooth identification module 1210, an adjacent tooth selection module 1220, and a tooth movement module 1230 may be stored in the memory 1200. However, not all of the modules shown in FIG. 2 are essential modules. In the memory 1200, more modules may be stored than the modules illustrated in FIG. 2, or fewer modules may be stored.

The "module" included in the memory 1200 may mean a unit for processing a function or operation performed by the at least one processor 1300. The "module" included in the memory 1200 may be implemented as software such as instructions, algorithms, data structures, or program codes.

In an embodiment, the tooth identification module 1210 may include instructions or program codes related to an operation or function of identifying a plurality of teeth based on an oral image acquired through the communication interface 1400. In an embodiment, the tooth identification module 1210 may further include instructions or program codes related to an operation or function of identifying the gingiva, dental arch line, and dental midline based on an oral image.

In an embodiment, the at least one processor 1300 may identify a plurality of teeth, gingiva, an dental arch line, and a dental midline from an oral image by executing the tooth identification module 1210.

In an embodiment, the adjacent tooth selection module 1230 may include instructions or program codes related to an operation or function of selecting at least one adjacent tooth adjacent to a selected one selected point based on a selection signal including information for selecting one selected point among a plurality of selection points.

In an embodiment, the adjacent tooth selection module 1230 may include instructions or program codes related to an operation or function of selecting, as at least one adjacent tooth, at least one of a second adjacent tooth adjacent in the mesial direction or a first adjacent tooth adjacent in the distal direction from the selected one selection point according to the position of the selected one selection point in the oral cavity.

In an embodiment, the at least one processor 1300 may select at least one adjacent tooth adjacent to the selected one selection point by executing the adjacent tooth selection module 1230.

In an embodiment, the tooth movement module 1230 may include instructions or program codes related to an operation or function of moving at least one adjacent tooth by a target movement amount in a direction toward a selected one selection point based on the acquired target movement amount. In an embodiment, as the at least one processor 1300 executes the tooth movement module 1230, the at least one adjacent tooth may be moved by a target movement amount in a direction toward a selected selection point.

In an embodiment, the tooth movement module 1230 may further include instructions or program codes related to an operation or function of moving at least one adjacent tooth in a direction toward a dental arch line. In an embodiment, as the at least one processor 1300 executes the tooth movement module 1230, at least one adjacent tooth may be moved by a target movement amount in a direction toward a selected selection point, and then moved in a direction toward a dental arch line.

In an embodiment, the tooth movement module 1230 may further include instructions or program codes related to an operation or function of moving a target tooth to be moved in a direction toward at least one adjacent tooth. In an embodiment, as the at least one processor 1300 executes the tooth movement module 1230, at least one adjacent tooth may be moved by a target movement amount in a direction toward a selected selection point, and then a target tooth to be moved may be moved in a direction toward at least one adjacent tooth.

However, the present disclosure is not limited thereto. In an embodiment, a module including instructions or program codes related to an operation or function of generating a selection signal in response to an operation of selecting at least one selection point from among a plurality of selection points may be further stored in the memory 1200.

In addition, a selection point calculation module including instructions or program codes related to an operation or function of calculating a plurality of selection points located between respective two adjacent teeth based on a plurality of teeth may be further stored in the memory 1200.

In an embodiment, the at least one processor 1300 may be configured with at least one of a central processing unit, a microprocessor, a graphic processing unit, an application processor (AP), application specific integrated circuits (ASICs), digital signal processors (DSPs), digital signal processing devices (DSPDs), programmable logic devices (PLDs), field programmable gate arrays (FPGAs), a neural processing unit, or artificial intelligence (AI) dedicated processors designed with a hardware structure specialized in learning and processing of AI, but is not limited thereto.

In an embodiment, the at least one processor 1300 may execute various types of modules stored in the memory 1200. In an embodiment, the at least one processor 1300 may execute the tooth identification module 1210, the adjacent tooth selection module 1230, and the tooth movement module 1230 stored in the memory 1200. In an embodiment, the at least one processor 1300 may execute at least one instruction constituting various types of modules stored in the memory 1200.

In an embodiment, the communication interface 1400 may perform data communication with an external server or at least one external electronic device (e.g., an oral scanner, a table scanner, or an external medical device) under the control by the at least one processor 1300. In an embodiment, the communication interface 1400 may perform data communication with a server or other peripheral electronic devices by using at least one of data communication methods including wired local area network (LAN), wireless LAN, Wi-Fi, Bluetooth, Zigbee, Wi-Fi direct (WFD), infrared data association (IrDA), Bluetooth low energy (BLE), near field communication (NFC), wireless broadband internet (Wibro), world interoperability for microwave access (WiMax), shared wireless access protocol (SWAP), wireless gigabit alliance (WiGig), and RF communication.

In an embodiment, the communication interface 1400 may include at least one port for being connected to an external electronic device by a wired cable in order to communicate with the external electronic device (e.g., an oral scanner, etc.) by wire. The communication interface 1400 may communicate with an external electronic device wiredly connected through at least one port.

In an embodiment, the user interface 1500 may receive a user input for controlling the oral image processing apparatus 1000. The user interface 1500 may include a user input device including a touch interface for sensing a user's touch, a button for receiving a user's push operation, a mouse or a keyboard for designating or selecting a point on a user interface screen, but is not limited thereto.

The user interface 1500 according to an embodiment may receive a user input for selecting one selection point from among a plurality of selection points. In addition, the user interface 1500 may receive a user input for inputting a target movement amount of at least one adjacent tooth.

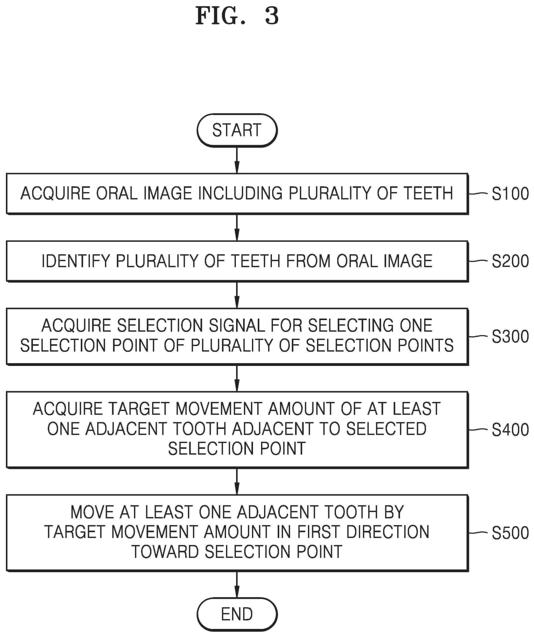

FIG. 3 is a flowchart for describing an oral image processing method according to an embodiment of the disclosure. The oral image processing method illustrated in FIG. 3 may be performed by the oral image processing apparatus 1000.

Referring to FIGS. 1, 2, and 3, in an embodiment, the oral image processing method may include an operation S100 of acquiring an oral image including a plurality of teeth 100, 110, and 120. In the operation S100 of acquiring an oral image, the at least one processor 1300 may acquire an oral image through the communication interface 1400. In an embodiment, the oral image may be an image acquired through a 3D scanner. The oral image may be an image stored in a library.

In an embodiment, the oral image processing method may include an operation S200 of identifying a plurality of teeth 100, 110, and 120 from the acquired oral image. In one embodiment, the operation S200 of identifying the plurality of teeth 100, 110, and 120 may further include an operation of identifying the gingiva, the dental midline, and the dental arch line. In the operation S200 of identifying a plurality of teeth 100, 110, and 120, the at least one processor 1300 may execute the tooth identification module 1210, to thereby identify a plurality of teeth 100, 110, 120, or identify a plurality of teeth 100, 110, and 120, gingiva, a dental midline, and a dental arch line. In an embodiment, the operation S100 of acquiring an oral image and the operation S200 of identifying a plurality of teeth 100, 110, and 120 from the oral image are shown separately in the oral image processing method in FIG. 3, but the present disclosure is not limited thereto. In an embodiment, the operation S100 of acquiring an oral image and the operation S200 of identifying a plurality of teeth 100, 110, and 120 from the oral image may be performed in one operation.

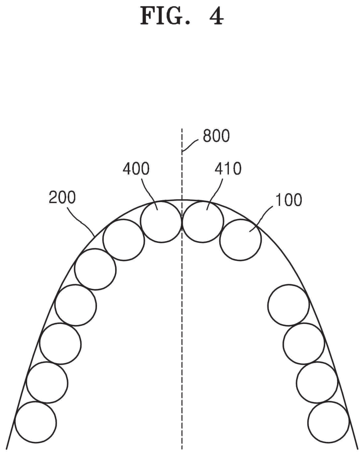

Hereinafter, the operation S200 of identifying a plurality of teeth 100, 110, and 120 will be described later with reference to FIG. 4.

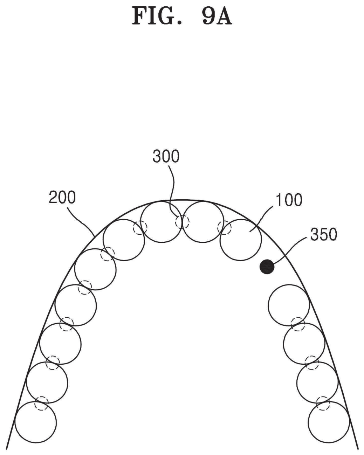

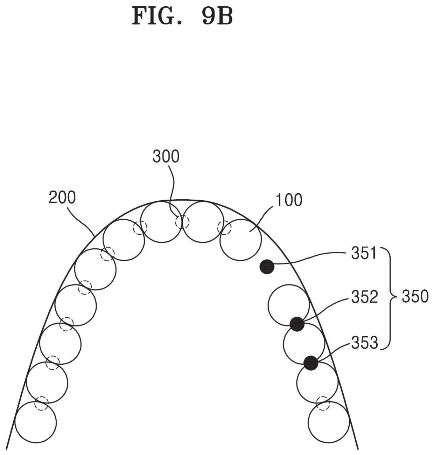

In an embodiment, the oral image processing method may include an operation S300 of acquiring a selection signal for selecting at least one selection point from among a plurality of selection points. In the operation S300 of acquiring the selection signal, the at least one processor 1300 may acquire a selection signal generated based on a user input provided through the user interface 1500. Hereinafter, the operation S300 of acquiring the selection signal will be described later with reference to FIGS. 9A and 9B.

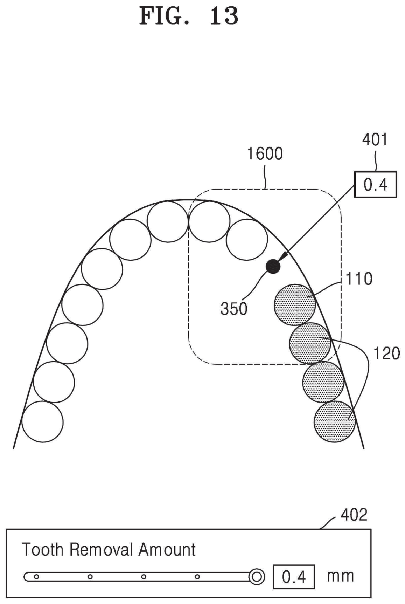

In an embodiment, the oral image processing method may include an operation S400 of acquiring a target movement amount of at least one adjacent tooth adjacent to the selected selection point. In the operation S400 of acquiring the target movement amount, the at least one processor 1300 may acquire the target movement amount through the user interface 1500. Hereinafter, the operation S400 of acquiring the target movement amount will be described later with reference to FIG. 13.

In an embodiment, the operation S300 of acquiring a selection signal and the operation S400 of acquiring a target movement amount are sequentially illustrated in FIG. 3, but the oral image processing method of the present disclosure is not limited thereto. In an embodiment, in the oral image processing method, the step of acquiring a selection signal may be performed after the step of acquiring the target movement amount.

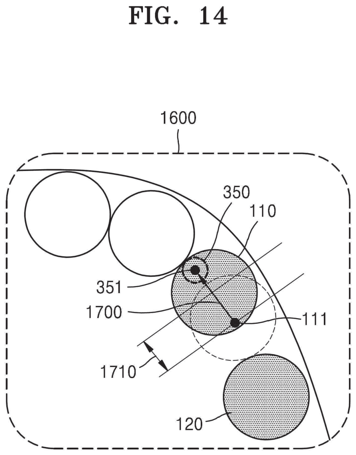

In an embodiment, the oral image processing method may include an operation S500 of moving at least one adjacent tooth by a target movement amount in a first movement direction toward the selection point, based on the target movement amount. In the operation S500 of moving at least one adjacent tooth by a target movement amount, the at least one processor 1300 may execute the tooth movement module 1230, to thereby move at least one adjacent tooth by a target movement amount in a direction toward the selection point. Hereinafter, the operation S500 of moving at least one adjacent tooth by a target movement amount will be described later with reference to FIG. 14.

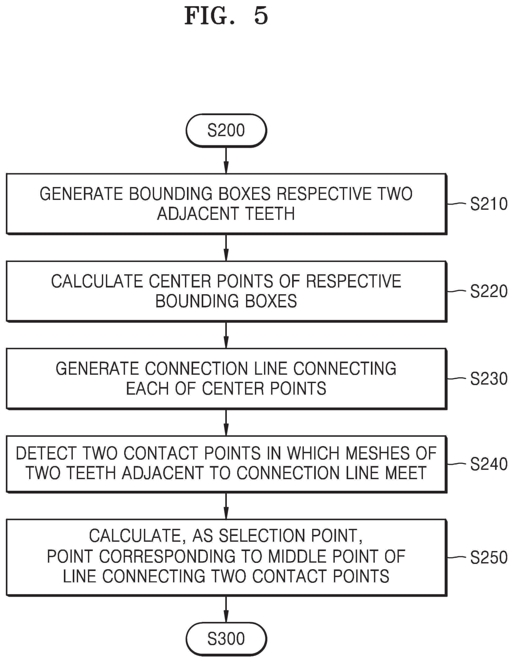

However, the present disclosure is not limited thereto. In an embodiment, the oral image processing method may further include an operation of calculating a plurality of selection points positioned between respective two adjacent teeth based on the identified plurality of teeth 100, 110, and 120. Hereinafter, the operation of calculating a plurality of selection points will be described with reference to FIGS. 5 to 8.

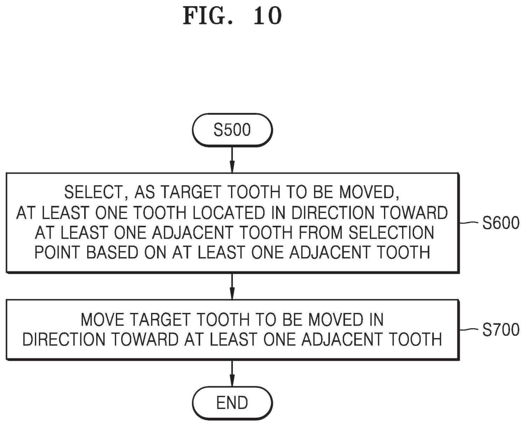

In addition, in an embodiment, the oral image processing method may further include an operation of selecting at least one adjacent tooth adjacent to the selected selection point. In an embodiment, the at least one processor 1300 may identify at least one selection point from among a plurality of selection points based on coordinate information of a user input included in the acquired selection signal, a region of the user input, an area and shape of the region, and the like. The at least one processor 1300 may select at least one adjacent tooth adjacent to the identified at least one selection point according to a predetermined criterion. Hereinafter, the operation S500 of selecting at least one adjacent tooth will be described with reference to FIGS. 10 to 11C.

FIG. 4 is a diagram for describing a plurality of teeth identified from an oral image according to an embodiment of the disclosure. Hereinafter, the same reference numerals are assigned to the same configuration as that described in FIG. 1, and redundant descriptions are omitted.

Referring to FIG. 4, a plurality of teeth 100, a dental arch line 200, and a dental midline 800 identified from an oral image are shown. In an embodiment, gingiva may be further identified from the oral image. In this case, the "dental midline" may be a line passing between the first tooth 400 on the left and the first tooth 410 on the right. Specifically, the dental midline may be a line passing between the first tooth of the left maxilla and the first tooth of the right maxilla, or a line passing between the first tooth of the left mandible and the first tooth of the right mandible.

In an embodiment, the number and arrangement of the plurality of teeth 100, the shape of the dental arch line 200, the position of the dental midline 800, and the like may be identified from the oral image. The at least one processor 1300 may perform an oral image processing operation to be described later based on the identified plurality of teeth 100, dental arch line 200, dental midline 800, and the like.

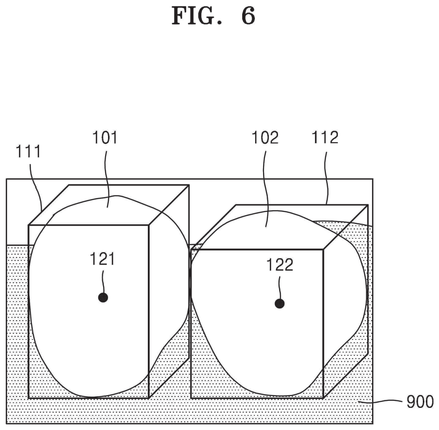

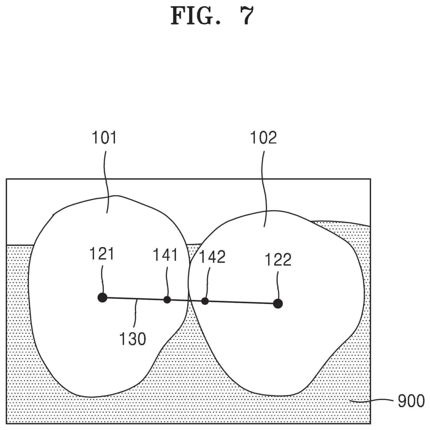

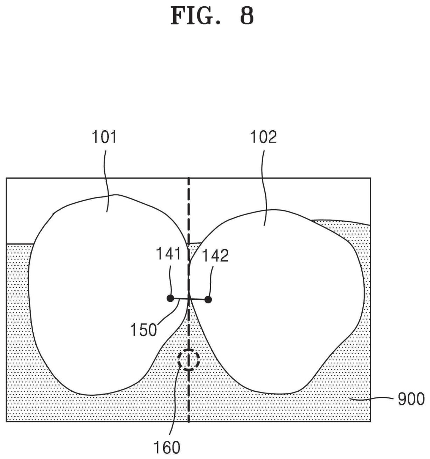

FIG. 5 is a flowchart for describing a method of calculating a plurality of selection points positioned between respective two adjacent teeth according to an embodiment of the disclosure. FIG. 6 is a diagram for describing a method of generating respective bounding boxes of two adjacent teeth according to an embodiment of the disclosure. FIG. 7 is a diagram for describing a method of calculating center points of respective bounding boxes and generating a connection line connecting the center points according to an embodiment of the disclosure. FIG. 8 is a diagram for describing a method for calculating a contact point and a selection point where a connection line and a mesh of two teeth meet, according to an embodiment of the disclosure. Hereinafter, the same reference numerals are assigned to the same configuration and operations as those described in FIGS. 1 and 3, and redundant descriptions are omitted.

Referring to FIGS. 5 and 6, the oral image processing method may further include an operation of calculating a plurality of selection points positioned between respective two adjacent teeth based on the identified plurality of teeth 100, 110, and 120.

In an embodiment, the operation of calculating a plurality of selection points may include an operation S210 of generating respective bounding boxes 111 and 112 of two adjacent teeth 101 and 102. Hereinafter, for convenience of description, only two adjacent teeth among a plurality of teeth are illustrated in FIGS. 6 to 8.

In an embodiment, the respective bounding boxes 111 and 112 of the two adjacent teeth 101 and 102 may be generated based on a mesh of each of the two adjacent teeth 101 and 102. A rectangular parallelepiped-shaped bounding box may be generated by connecting a point having a maximum coordinate value of a mesh constituting two adjacent teeth 101 and 102 and a point having a minimum coordinate value thereof to each other. In an embodiment, the bounding box includes all the meshes constituting each of the teeth 101 and 102, and may mean a box having the smallest size.

In an embodiment, the step of calculating the plurality of selection points may include an operation S220 of calculating the respective center points 121 and 122 of the bounding boxes 111 and 112. In an embodiment, the respective center points 121 and 122 of the bounding boxes 111 and 112 may be defined as diagonal centers of the respective bounding boxes 111 and 112.

Referring to FIGS. 5 and 7, in an embodiment, the step of calculating a plurality of selection points may include an operation S230 of generating a connection line 130 connecting the calculated center points 121 and 122 to each other. In an embodiment, the connection line 130 may mean a line generated to pass all of the two center points 121 and 122 included in the respective bounding boxes 111 and 112.

In an embodiment, the step of calculating the plurality of selection points may include an operation S240 of detecting two contacts 141 and 142 in which the respective meshes of the two teeth 101 and 102 adjacent to the connection line 130 meet. In an embodiment, the two contacts 141 and 142 may be points at which a mesh of each of the two teeth 101 and 102 and the connection line 130 intersect.

Referring to FIGS. 5 and 8, in an embodiment, the step of calculating a plurality of selection points may include an operation S350 of calculating a point 160 corresponding to a middle point of the line 150 connecting the two contact points to each other as one selection point 160 among the plurality of selection points.

In an embodiment, the line 150 connecting the two contacts to each other may be a line generated to pass through the two contacts. In one embodiment, the operation S350 of calculating one selection point 160 from among the plurality of selection points may further include an operation of calculating one selection point 160 by projecting a point corresponding to the middle point of the line 150 connecting the two contact points onto gingiva 900.

FIG. 9A is a diagram for describing selection of one selection point from among a plurality of selection points according to an embodiment of the disclosure. FIG. 9B is a diagram for describing selection of two or more selection points from among a plurality of selection points according to an embodiment of the disclosure. Hereinafter, the same reference numerals are assigned to the same configuration as those described in FIGS. 1 and 4, and redundant descriptions are omitted.

Referring to FIGS. 1, 3, and 9A, a plurality of teeth 100, an dental arch line 200, a plurality of selection points 300, and one selection point 350 selected based on a selection signal among the plurality of selection points 300 are shown in FIG. 9A.

In an embodiment, the selection signal may be a signal generated based on an operation in which the user clicks on at least one selection point 350 of a plurality of selection points 300 through the user interface 1500 or an operation in which at least one selection point 350 is selected through drag.

In an embodiment, FIG. 9A illustrates that the selected selection point 350 corresponds to a point located between two teeth with a relatively large spacing between two adjacent teeth among the plurality of teeth 100. In an embodiment, the user may view a result of the movement of at least one adjacent tooth adjacent to the corresponding selection point through an operation of selecting a selection point corresponding to a place having a large gap between two adjacent teeth among a plurality of teeth 100 identified based on the acquired oral image and a plurality of calculated selection points 300.

Referring to FIGS. 1, 3, and 9B, a plurality of teeth 100, an dental arch line 200, a plurality of selection points 300, and three selection points 351, 352, and 353 identified based on a selection signal among the plurality of selection points 300 are shown in FIG. 9B.

In an embodiment, the selection signal may be a signal generated based on an operation in which the user selects three selection points 351, 352, and 353 among the plurality of selection points 300. The selection signal may be a signal generated by an operation of the user continuously clicking on three selection points 350 among the plurality of selection points 300 or selecting the three selection points 350 through drag.

In FIGS. 9A and 9B, one selected from among the at least one selection points is shown to correspond to a point between two teeth with a relatively large spacing between adjacent teeth among the plurality of teeth 100. However, the present disclosure is not limited thereto. The user may select at least one selection point around the at least one adjacent tooth to be moved among the plurality of selection points 300 regardless of the gap between adjacent teeth, and the oral image processing apparatus 1000 may identify the at least one selection point based on the selection signal generated by the corresponding operation.

In addition, as illustrated in FIG. 9B, the selected at least one selection point may include a selection point corresponding to a position where a gap between adjacent teeth is relatively larger than gaps between other teeth, and other selection points adjacent to the selection point.

Hereinafter, for convenience of description, it will be described that one selection point 350 is selected from among the plurality of selection points 300 based on the selection signal.

FIG. 10 is a flowchart for describing a method of selecting a target tooth to be moved and moving the selected target tooth to be moved, according to an embodiment of the disclosure. Hereinafter, the same reference numerals are assigned to the same operations as those described in FIG. 3, and redundant descriptions are omitted.

Referring to FIGS. 2, 3, and 10, the oral image processing method may include an operation S600 of selecting, as a target tooth to be moved, at least one tooth located in a direction toward at least one adjacent tooth from the selection point based on the at least one adjacent tooth. In an embodiment, the operation S600 of selecting at least one tooth as a target tooth to be moved may be performed after the operation S500 of moving at least one adjacent tooth in the first moving direction.

In the operation S600 of selecting at least one tooth as a target tooth to be moved, the at least one processor 1300 may select, as a target tooth to be moved, at least one tooth positioned in a direction toward at least one adjacent tooth from the selection point based on the at least one adjacent tooth.

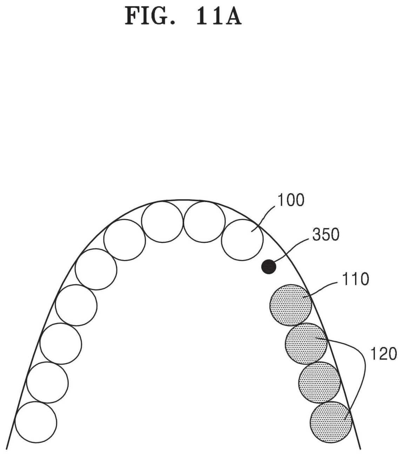

Hereinafter, the operation S600 of selecting at least one tooth as a target tooth to be moved will be described later with reference to FIGS. 11A to 11C.

In an embodiment, the oral image processing method may include an operation S700 of moving the target tooth to be moved in a direction toward at least one adjacent tooth.

In the operation S700 of moving the target tooth to be moved in a direction toward at least one adjacent tooth, the at least one processor 1300 may move the target tooth to be moved in a direction toward the at least one adjacent tooth. Therefore, it is possible to prevent the gap between the at least one adjacent tooth and the target tooth to be moved from widening compared to before moving at least one adjacent tooth.

In an embodiment, a direction toward at least one adjacent tooth may be a direction from a center point of a bounding box of a target tooth to be moved toward a center point of a bounding box of at least one adjacent tooth.

In an embodiment, the oral image processing method may include an operation of moving the target tooth to be moved in a direction toward the dental arch line after the operation S700 of moving the target tooth to be moved in a direction toward at least one adjacent tooth.

In an embodiment, as the target tooth to be moved is moved toward at least one adjacent tooth, a gap between the target tooth to be moved and the dental arch line may vary. Therefore, by moving the target tooth to be moved in a direction toward the dental arch line, a gap between the target tooth to be moved and the dental arch line may be maintained.

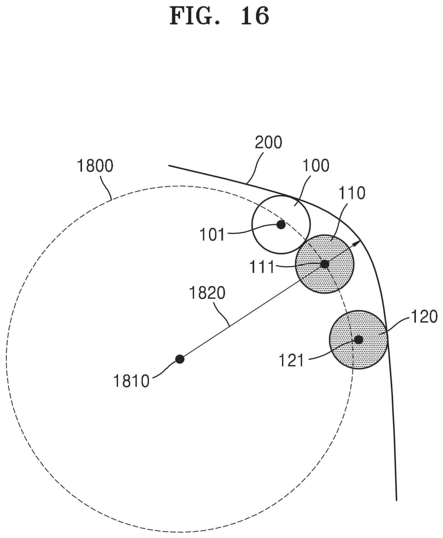

Hereinafter, the direction toward the dental arch line will be described later with reference to FIG. 16.

FIG. 11A is a diagram for describing selecting a first adjacent tooth and a target tooth to be moved corresponding to the first adjacent tooth according to an embodiment of the disclosure. Hereinafter, the same reference numerals are assigned to the same configuration as those described in FIGS. 4 and 9A, and redundant descriptions are omitted.

Referring to FIGS. 2 and 11A, in an embodiment, the at least one adjacent tooth may include at least one of a first adjacent tooth adjacent in a distal direction or a second adjacent tooth adjacent in a mesial direction from the selected one selection point.

In an embodiment, when at least one adjacent tooth includes the first adjacent tooth, in the operation of selecting the target tooth to be moved, the first mobile tooth located in the distal direction based on the first adjacent tooth may be selected as the target tooth to be moved.

In an embodiment, when the first adjacent tooth 110 in the distal direction from the selection point 350 is selected as the adjacent tooth of the selection point 350, the at least one processor 1300 may select, as a target tooth to be moved, the first mobile tooth 120 located in the distal direction from the first adjacent tooth 110 among the plurality of teeth. In an embodiment, the at least one processor 1300 may select, as target teeth to be moved, all teeth 120 located in the distal direction from the first adjacent tooth 110.

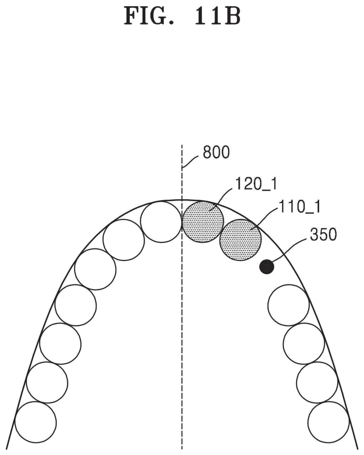

FIG. 11B is a diagram for describing selecting a second adjacent tooth and a target tooth to be moved corresponding to the second adjacent tooth according to an embodiment of the disclosure. Hereinafter, the same reference numerals are assigned to the same configuration as those described in FIGS. 4 , 9A, and 11A, and redundant descriptions are omitted.

Referring to FIGS. 2 and 11B, in an embodiment, when at least one adjacent tooth includes the first adjacent tooth, in the operation of selecting the target tooth to be moved, the first mobile tooth located in the distal direction up to the dental midline based on the first adjacent tooth may be selected as a target tooth to be moved. The at least one processor 1300 may select a second adjacent tooth 110_1 adjacent to the selection point 350 in the mesial direction from the identified one selection point 350 as an adjacent tooth of the selection point 350.

In an embodiment, when a second adjacent tooth 110_1 in the mesial direction from the selection point 350 is selected as the adjacent tooth of the selection point 350, the at least one processor 1300 may select, as a target tooth to be moved, a second mobile tooth 120_1 located in the mesial direction from the second adjacent tooth 110_1 among the plurality of teeth. In an embodiment, the at least one processor 1300 may select, as target teeth to be moved, all teeth 120_1 located in the mesial direction up to the dental midline 800 based on the second adjacent tooth 110_1.

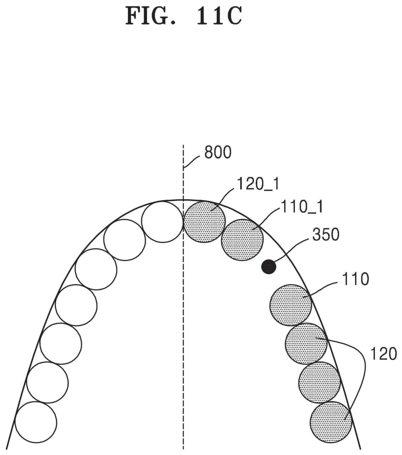

FIG. 11C is a diagram for describing selecting a first adjacent tooth and a second adjacent tooth and a target tooth to be moved corresponding to the first adjacent tooth and the second adjacent tooth, according to an embodiment of the disclosure. Hereinafter, the same reference numerals are assigned to the same configuration as those described in FIGS. 4 , 9A, 11A, and 11B, and redundant descriptions are omitted.

Referring to FIGS. 2 and 11C, in an embodiment, when at least one adjacent tooth includes a first adjacent tooth and a second adjacent tooth, in the operation of selecting a target tooth to be moved, first and second mobile teeth may be selected as target teeth to be moved. The at least one processor 1300 may select a first adjacent tooth 110 adjacent to the distal direction and a second adjacent tooth 110_1 adjacent to the mesial direction from the identified one selection point 350 as adjacent teeth of the selection point 350.

In an embodiment, when the first adjacent tooth 110 and the second adjacent tooth 110_1 of the selection point 350 are selected as adjacent teeth of the selection point 350, the at least one processor 1300 may select, as target teeth to be moved, the first mobile tooth 120 located in the distal direction from the first adjacent tooth 110 and the second mobile tooth 120_1 located in the mesial direction from the second adjacent tooth 110_1 among a plurality of teeth. In an embodiment, the at least one processor 1300 may select, as target teeth to be moved, all teeth 120 located in a distal direction from the first adjacent tooth 110 and all teeth 120_1 located in a mesial direction up to the dental midline 800 based on the second adjacent tooth 110_1.

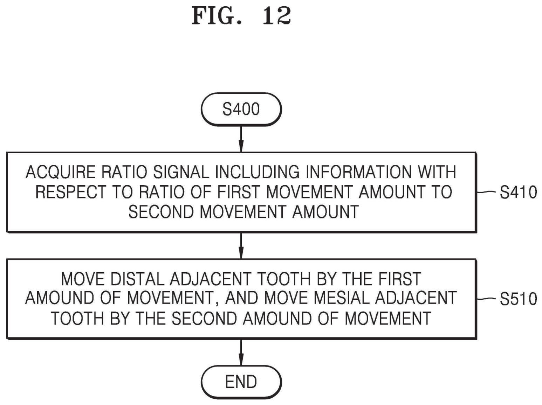

FIG. 12 is a flowchart for describing a movement amount of each of a first adjacent tooth and a second adjacent tooth when a first adjacent tooth and a second adjacent tooth are selected according to an embodiment of the disclosure. Hereinafter, the same reference numerals are assigned to the same operations as those described in FIGS. 3 and 10, and redundant descriptions are omitted.

Referring to FIGS. 3, 10, 11C, and 12, in an embodiment, when at least one adjacent tooth includes the first adjacent tooth 110 and the second adjacent tooth 110_1, the oral image processing method may include an operation S510 of moving the first adjacent tooth 110 to the selected selection point 350 by a first movement amount and moving the second adjacent tooth 120 to the selected selection point 350 by a second movement amount.

In an embodiment, the oral image processing method may include an operation S410 of acquiring a ratio signal including information on a ratio between the first movement amount and the second movement amount.

In an embodiment, in the operation S410 of acquiring the ratio signal, the at least one processor 1300 may acquire a ratio signal generated based on a user input provided through the user interface 1500. In an embodiment, the ratio signal may be a signal generated based on an operation of inputting a ratio of a first movement amount for moving the first adjacent tooth 110 and a second movement amount for moving the second adjacent tooth 110_1 among target movement amounts by the user interface 1500.

However, the present disclosure is not limited thereto. In an embodiment, the ratio of the first movement amount to the second movement amount may be a preset ratio. The ratio of the first movement amount to the second movement amount may be preset by a user using the oral image processing apparatus 1000 (see FIG. 2).

In addition, the ratio of the first movement amount to the second movement amount may be determined according to the distance from the dental midline 800 to the selected selection point 350. In an embodiment, the ratio of the first movement amount to the second movement amount may be proportional to the distance from the dental midline 800 to the selected selection point 350. In an embodiment, the ratio of the first movement amount to the second movement amount may be determined according to the tooth number of two adjacent teeth in which the selected selection point 350 is located. In an embodiment, as the tooth number of two adjacent teeth increases, the second movement amount may be greater than the first movement amount. In an embodiment, as the tooth number of two adjacent teeth decreases, the first movement amount may be greater than the second movement amount.

In an embodiment, a sum of the first movement amount and the second movement amount may be equal to a target movement amount.

FIG. 13 is a diagram for describing a target movement amount of at least one adjacent tooth according to an embodiment of the disclosure. Hereinafter, the same reference numerals are assigned to the same configuration as those described in FIGS. 1 and 11A, and redundant descriptions are omitted.

Referring to FIGS. 2 and 13, at least one adjacent tooth 110 adjacent to at least one selection point 350 selected from a plurality of selection points and target teeth 120 to be moved are shown. The at least one processor 1300 may control the display 1100 to display user input interfaces 401 and 402 capable of inputting a target movement amount of the at least one adjacent tooth 110 in response to the selected at least one selection point 350.

In an embodiment, the user may input a target movement amount into the user input interfaces 401 and 402 through the user interface 1500. In an embodiment, the at least one processor 1300 may acquire a numerical signal including information on the target movement amount generated based on an input of the target movement amount input to the user input interfaces 401 and 402.

In an embodiment, the user input interfaces may include a first user input interface 401 and a second user input interface 402. In an embodiment, the first user input interface 401 may be an interface that provides an input space through which a user may input a number corresponding to a target movement amount through a user interface such as a keyboard. In an embodiment, the second user input interface 402 may be an interface that provides a sliding bar that a user may move to correspond to a target movement amount through a user interface such as a mouse. However, the present disclosure is not limited thereto, and the user may input a target movement amount by inputting a number into the input space of the first user input interface 401 through the touch interface, or may input a target movement amount by moving the sliding bar of the second user input interface 402.

In addition, FIG. 13 shows that one selection point 350 of a plurality of selection points has been selected, but the present disclosure is not limited thereto. When two or more selection points are selected among a plurality of selection points, in an embodiment, a number input to the first user input interface may be set as a target movement amount of two or more adjacent teeth adjacent to each selection point. In addition, two or more first user input interfaces corresponding to two or more selection points, respectively, may be displayed.

In an embodiment, the input target movement amount according to the movement of the sliding bar of the second user input interface may be set as the target movement amount of two or more adjacent teeth adjacent to each selection point.

FIG. 14 is a diagram for describing moving at least one adjacent tooth to an identified selection point by a target movement amount, according to an embodiment of the disclosure. Hereinafter, the same reference numerals are assigned to the same configuration as that described in FIG. 11A, and redundant descriptions are omitted.

Referring to FIGS. 2, 13, and 14, for convenience of description, a partial area 1600 of FIG. 13 is shown in FIG. 14.

In an embodiment, a plurality of teeth, a single selection point 350 selected based on a selection signal, a first adjacent tooth 110, and a target tooth 120 to be moved are shown in FIG. 14.

In an embodiment, the at least one processor 1300 may move the first adjacent tooth 110 by a target movement amount in a direction 1700 toward one selection point 350. In an embodiment, the direction 1700 toward one selection point 350 may be a direction toward one selection point 350 from the first adjacent tooth 110. In an embodiment, the direction 1700 toward one selection point 350 may be a direction toward the center point 351 of one selection point 350 from a point where the center point 111 of the bounding box of the first adjacent tooth 110 is projected onto the gingiva 900 (see FIG. 8).

In an embodiment, the at least one processor 1300 may move the first adjacent tooth 110 by a final movement amount 1710 in a direction 1700 toward one selection point 350. The at least one processor 1300 may move the first adjacent tooth 110 in the direction 1700 toward one selection point 350 until the difference between the final movement amount 1710 and the target movement amount is equal to or less than a preset first reference error. In this case, as the first reference error is set to be smaller, the accuracy of the operation of moving the first adjacent tooth 110 by the target movement amount may be increased. As the first reference error is set to be larger, the speed of the operation of moving the first adjacent tooth 110 by the target movement amount may be increased.

In an embodiment, only the operation of moving the first adjacent tooth 110 in the direction 1500 toward one selection point 350 is illustrated in FIG. 14, but the present disclosure is not limited thereto. The at least one processor 1300 may move the first adjacent tooth 110 in the direction 1700 toward one selection point 350 and then move the target tooth 120 to be moved in the direction toward the first adjacent tooth 110. Accordingly, it is possible to prevent the gap between the target tooth 120 to be moved and the first adjacent tooth 110 from widening. In this case, the direction toward the first adjacent tooth 110 may be a direction toward the point at which the center point 111 of the first adjacent tooth 110 is projected to the gingival 900 from the center point of the bounding box of the target tooth 120 to be moved is set to the gingival 900.

In addition, for convenience of description, FIG. 14 illustrates the movement operation of the first adjacent tooth 110 and the target tooth 120 to be moved adjacent to each other in the distal direction at one selection point 350, but the disclosure may also be applied to the movement operation of the second adjacent tooth and the target tooth to be moved adjacent to each other in the mesial direction at one selection point 350.

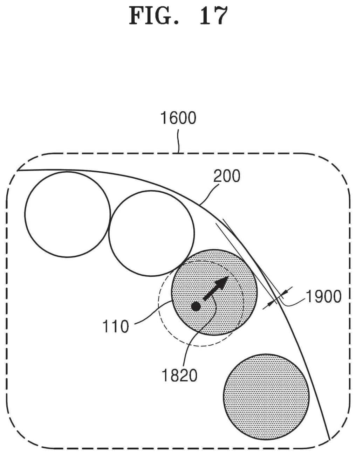

FIG. 16 is a flowchart for describing moving at least one adjacent tooth to a dental arch line according to an embodiment of the disclosure. FIG. 17 is a diagram for describing a direction in which at least one adjacent tooth is moved to a dental arch line according to an embodiment of the disclosure.

Hereinafter, the same reference numerals are assigned to the same configuration and operations as those described in FIGS. 3 and 10, and redundant descriptions are omitted.

Referring to FIGS. 2, 3, 15, and 16, the oral image processing method may include an operation S260 of acquiring an dental arch line 200 corresponding to a plurality of teeth from an oral image. The oral image processing method may include an operation S520 of moving at least one adjacent tooth 110 moved in a first movement direction in a second movement direction 1820 toward the dental arch line 200. However, the present disclosure is not limited thereto, and the operation of acquiring the dental arch line 200 from an oral image and the operation of identifying a plurality of teeth may be performed in a single operation.

In an embodiment, the oral image processing method may include the operation S520 of moving the at least one adjacent tooth 110 in a second movement direction 1820 after the operation S500 of moving the at least one adjacent tooth 110 by a target movement amount in the first movement direction.

In an embodiment, in the step of moving the at least one adjacent tooth 110 in the first moving direction, a distance between the dental arch line 200 and the at least one adjacent tooth 110 may be changed. In an embodiment, as the at least one adjacent tooth 110 is moved in the first moving direction, a distance between the at least one adjacent tooth 110 and the dental arch line 200 may increase. Accordingly, by moving at least one adjacent tooth 110 in the second movement direction 1820, the distance between at least one adjacent tooth 110 and the dental arch line 200 may be maintained as the distance before moving at least one adjacent tooth 110 in the direction toward at least one selection point 350.

In an embodiment, the second movement direction 1820 may be calculated using a total of three teeth including two teeth adjacent to both sides of the at least one adjacent tooth 110, based on the at least one adjacent tooth 110. Hereinafter, for convenience of description, one adjacent tooth 110 is illustrated in FIG. 17.

In an embodiment, the at least one processor 1300 may identify two teeth 100 and 120 located on both sides of one adjacent tooth 110, based on the one adjacent tooth 110. The at least one processor 1300 may calculate a first center point 111 of the bounding box of the one adjacent tooth 110 and respective second and third center points 101 and 121 of the bounding boxes of the two teeth 100 and 120. The at least one processor 1300 generates a virtual circle 1800 passing through a total of three orthogonal projected points after respective orthogonal projections of the calculated first to third center points 111, 101, and 121 onto the gingiva 900 (see FIG. 8).

In an embodiment, the at least one processor 1300 may calculate, as the second movement direction 1820, the direction from the center point 1810 of the generated virtual circle 1800 toward the dental arch line 200. In this case, the at least one processor 1300 may orthogonally project the dental arch line 200 onto the gingiva 900, and then calculate, as the second movement direction 1820, the direction from the center point 1810 of the virtual circle 1800 toward the dental arch line orthogonally projected in the gingiva 900.

FIG. 17 is a diagram for describing that at least one adjacent tooth is moved to a dental arch line according to an embodiment of the disclosure. Hereinafter, the same reference numerals are assigned to the same configuration as those described in FIGS. 11A and 16, and redundant descriptions are omitted.

Referring to FIGS. 2, 13, and 17, for convenience of description, a partial area 1600 of FIG. 13 is shown in FIG. 18.

In an embodiment, in FIG. 17, a plurality of teeth and a first adjacent tooth 110 are shown. In an embodiment, the at least one processor 1300 may move the first adjacent tooth 110 in the second movement direction 1820 after moving the first adjacent tooth 110 in the first movement direction by a target movement amount.

In an embodiment, the at least one processor 1300 may move the first adjacent tooth 110 in the second movement direction 1820. The at least one processor 1300 may move the first adjacent tooth 110 in the second movement direction 1820 until the distance 1900 between the first adjacent tooth 110 and the dental arch line 200 is equal to or less than a preset second reference error. In this case, as the second reference error is set smaller, the accuracy of the operation of maintaining the distance between the first adjacent tooth 110 and the dental arch line 200 may be increased. As the second reference error is set larger, the speed of the operation of maintaining the distance between the first adjacent tooth 110 and the dental arch line 200 may be increased.

In an embodiment, referring to FIGS. 10, 14, 15, and 17, the oral image processing method may repeat the step of moving at least one adjacent tooth 110 in the second movement direction 1820 so that a final movement amount 1710 in which at least one adjacent tooth 110 moves in a first movement direction 1700 is equal to or less than the first reference error, and a distance 1900 between the at least one adjacent tooth 110 and the dental arch line 200 is equal to or less than the second reference error.

Accordingly, the oral image processing apparatus 1000 may provide the user with an oral image in which at least one adjacent tooth 110 is moved to at least one selection point 350 by a target movement amount input within an error range, and the distance between the at least one adjacent tooth 110 and the dental arch line 200 is maintained within the error range.

The oral image processing method according to an embodiment may be implemented in the form of program instructions that may be performed through various computer means and recorded in a computer-readable medium. In addition, an embodiment of the present disclosure may be a computer-readable storage medium in which one or more programs including at least one instruction executing a method of processing an oral image are recorded.

The computer-readable storage medium may include program instructions, data files, data structures, and the like, alone or in combination. Here, examples of computer-readable storage media may include magnetic media such as hard disks, floppy disks and magnetic tapes, optical media such as CD-ROMs and DVDs, magneto-optical media such as floptical disks, and hardware devices configured to store and execute program instructions, such as ROMs, RAMs, and flash memories.

Here, a device-readable storage medium may be provided in the form of a non-transitory storage medium. Here, the "non-transitory storage medium" may mean a device in which the storage medium is tangible. In addition, the "non-transitory storage medium" may include a buffer in which data is temporarily stored.

The oral image processing method according to various embodiments disclosed in this document may be provided by being included in a computer program product. Computer program products may be distributed in the form of device-readable storage media (e.g., compact disk read only memory (CD-ROM). Alternatively, computer program products may be distributed online (e.g., downloaded or uploaded) directly through an application store (e.g., a play store, etc.) or between two user devices (e.g., smartphones). Specifically, a computer program product according to the disclosed embodiment may include a storage medium in which a program including at least one instruction is recorded to perform a method of processing an oral image according to the disclosed embodiment.

Although the embodiments have been described in detail above, the scope of the present disclosure is not limited thereto, and various modifications and improvements of those skilled in the art using the basic concepts of the present disclosure defined in the following claims also fall within the scope of the present disclosure.

## Claims

1. An oral image processing method comprising:
acquiring an oral image including a plurality of teeth;
acquiring a selection signal for selecting at least one selection point among a plurality of selection points positioned between respective two adjacent teeth among the plurality of teeth;
acquiring a target movement amount of at least one adjacent tooth adjacent to the selected selection point; and
moving the at least one adjacent tooth by the target movement amount in a first movement direction, which is a direction toward the selected selection point.

2. The oral image processing method of claim 1, further comprising:
providing a user input interface for inputting the target movement amount; and
acquiring the target movement amount through the user input interface.

3. The oral image processing method of claim 1, further comprising:
selecting, as a tooth to be moved, at least one tooth located in a direction from the selected at least one selection point toward the at least one adjacent tooth on the basis of the at least one adjacent tooth; and
moving the target tooth to be moved toward the at least one adjacent tooth.

4. The oral image processing method of claim 3, wherein the at least one adjacent tooth includes at least one of a first adjacent tooth adjacent in a distal direction or a second adjacent tooth adjacent in a mesial direction from the selected at least one selection point.

5. The oral image processing method of claim 4, further comprising:
when the at least one adjacent tooth includes the first adjacent tooth,
selecting, as the target tooth to be moved, a first mobile tooth located in the distal direction based on the first adjacent tooth;
when the at least one adjacent tooth includes the second adjacent tooth,
selecting, as the target tooth to be moved, a second mobile tooth located in the mesial direction to the dental midline based on the second adjacent tooth; and
when the at least one adjacent tooth includes the first adjacent tooth and the second adjacent tooth,
selecting the first mobile tooth and the second mobile tooth as the target teeth to be moved.

6. The oral image processing method of claim 4, further comprising:
when the at least one adjacent tooth includes the first adjacent tooth and the second adjacent tooth,
moving the first adjacent tooth to the selected at least one selection point by a first movement amount, and moving the second adjacent tooth to the selected at least one selection point by a second movement amount, wherein
a sum of the first movement amount and the second movement amount is equal to the target movement amount.

7. The oral image processing method of claim 6, wherein a ratio of the first movement amount to the second movement amount is a preset ratio, or the ratio of the first movement amount to the second movement amount is proportional to a distance from the dental midline to the at least one selected selection point.

8. The oral image processing method of claim 3, further comprising displaying a color of at least one adjacent tooth among the plurality of teeth and a color of the target tooth to be moved to be distinguished from the colors of the remaining teeth among the plurality of teeth.

9. The oral image processing method of claim 1, further comprising:
acquiring a dental arch line corresponding to the plurality of teeth from the oral image; and
moving the at least one adjacent tooth moved in the first moving direction in a second moving direction which is a direction toward the dental arch line.

10. The oral image processing method of claim 9, further comprising repeating moving the at least one adjacent tooth in the first movement direction and moving the at least one adjacent tooth in the second movement direction so that the at least one adjacent tooth is positioned close to the dental arch line.

11. An oral image processing apparatus comprising:
a memory storing at least one instruction; and
at least one processor executing the at least one instruction stored in the memory, wherein
the at least one processor is configured to:
acquire an oral image including a plurality of teeth;
acquire a selection signal for selecting at least one selection point among a plurality of selection points positioned between respective two adjacent teeth among the plurality of teeth;
acquire a target movement amount of at least one adjacent tooth adjacent to the selected selection point; and
move the at least one adjacent tooth by the target movement amount in a first movement direction, which is a direction toward the selected selection point.

12. The oral image processing apparatus of claim 11, wherein the at least one processor is configured to:
provide a user with a user input interface for inputting the target movement amount; and
acquire the target movement amount through the user input interface.

13. The oral image processing apparatus of claim 11, wherein the at least one processor is configured to:
select, as a target tooth to be moved, at least one tooth located in a direction from the selected at least one selection point toward the at least one adjacent tooth on the basis of the at least one adjacent tooth; and
move the target tooth to be moved toward the at least one adjacent tooth.

14. The oral image processing apparatus of claim 13, wherein the at least one adjacent tooth includes at least one of a first adjacent tooth adjacent in a distal direction or a second adjacent tooth adjacent in a mesial direction from the selected at least one selection point.

15. The oral image processing apparatus of claim 14, wherein,
when the at least one adjacent tooth includes the first adjacent tooth, the at least one processor is configured to select, as the target tooth to be moved, a first mobile tooth located in the distal direction based on the first adjacent tooth;
when the at least one adjacent tooth includes the second adjacent tooth, the at least one processor is configured to select, as the target tooth to be moved, a second mobile tooth located in the mesial direction to the dental midline based on the second adjacent tooth; and
when the at least one adjacent tooth includes the first adjacent tooth and the second adjacent tooth, the at least one processor is configured to select the first mobile tooth and the second mobile tooth as the target teeth to be moved.

16. The oral image processing apparatus of claim 14, wherein,
when the at least one adjacent tooth includes the first adjacent tooth and the second adjacent tooth,
the at least one processor is configured to:
move the first adjacent tooth to the selected at least one selection point by a first movement amount, and move the second adjacent tooth to the selected at least one selection point by a second movement amount, wherein
a sum of the first movement amount and the second movement amount is equal to the target movement amount.

17. The oral image processing apparatus of claim 16, wherein a ratio of the first movement amount to the second movement amount is a preset ratio, or the ratio of the first movement amount to the second movement amount is proportional to a distance from the dental midline to the at least one selected selection point.

18. The oral image processing apparatus of claim 11, wherein the at least one processor is configured to:
acquire a dental arch line corresponding to the plurality of teeth from the oral image; and
move the at least one adjacent tooth moved in the first moving direction in a second moving direction, which is a direction toward the dental arch line.

19. The oral image processing apparatus of claim 18, wherein the at least one processor is configured to repeat moving the at least one adjacent tooth in the first movement direction and moving the at least one adjacent tooth in the second movement direction so that the at least one adjacent tooth is positioned close to the dental arch line.

20. A non-transitory computer-readable recording medium having recorded thereon at least one instruction to be executed by at least one processor of an oral image processing apparatus,
wherein, when the at least one instruction is executed by the at least one processor of the oral image processing apparatus, the oral image processing apparatus is configured to:
acquire an oral image including a plurality of teeth;
acquire a selection signal for selecting at least one selection point among a plurality of selection points positioned between respective two adjacent teeth among the plurality of teeth;
acquire a target movement amount of at least one adjacent tooth adjacent to the selected selection point; and
move the at least one adjacent tooth by the target movement amount in a first movement direction, which is a direction toward the selected selection point.
